# EUROPÄISCHE PATENTSCHRIFT

(11) **EP 1 768 945 B1**
(45) Veröffentlichungstag und Bekanntmachung des Hinweises auf die Patenterteilung: **18.03.2009**
(21) Anmeldenummer: 05758124.1
(22) Anmeldetag: 06.07.2005
(51) Int. Cl.: C07C 49/84, C07C 49/835, C07D 311/22, A61K 31/12, A61P 31/04, A61P 35/00, C07C 45/61

(54) **SUBSTITUIERTE HYDROXYACETOPHENONDERIVATIVE**
SUBSTITUTED HYDROXYACETOPHENON DERIVATIVES
DERIVES D'HYDROXYACETOPHENONE SUBSTITUES

(30) Priorität: 06.07.2004 DE 102004032711
(43) Veröffentlichungstag der Anmeldung: 04.04.2007
(73) Patentinhaber: RIEMSER Arzneimittel AG, 17493 Greifswald - Insel Riems (DE)
(72) Erfinder: QUINCOCES SUAREZ, Jose Augustin, CEP, 01226-030 Sao Paulo (BR); PESEKE, Klaus, 18107 Elmenhorst/Lichtenhagen (DE); ESTRADA ROGER, Ernesto, 15892 Santiago de Compostella (ES)
(74) Vertreter: Vossius & Partner
(86) Internationale Anmeldenummer: PCT/EP2005/007307
(87) Internationale Veröffentlichungsnummer: WO 2006/003010

(56) Entgegenhaltungen:
- MITSUO TAKASUGI, TAKAHIRO MASUDA: "Three 4'-Hydroxyacetophenone-related phytoalexins from Polymnia Sonchifolia" PHYTOCHEMISTRY, Bd. 43, Nr. 5, 1996, Seiten 1019-1021, XP002348795
- F. TOMAS-BARBERAN ET AL.: "Antimicrobial phenolic compounds from three spanish helichrysum species" PHYTOCHEMISTRY, Bd. 29, Nr. 4, 1990, Seiten 1093-1095, XP002348796
- DATABASE CAPLUS [Online] CHEMICAL ABSTRACTS SERVICE, COLUMBUS, OHIO, US; J. L. RIOS ET AL.: "Isolation and identification of the antibacterial compounds from Helichrysum stoechas" XP002348844 gefunden im STN Database accession no. 115:197790 & JOURNAL OF ETHNOPHARMACOLOGY, Bd. 33, Nr. 1, 1991, Seiten 51-5,
- PARMAR V S ET AL: "Synthesis, Characterisation and In Vitro Anti-invasive Activity Screening of Polyphenolic and Heterocyclic Compounds" BIOORGANIC & MEDICINAL CHEMISTRY, ELSEVIER SCIENCE LTD, GB, Bd. 11, Nr. 6, 2003, Seiten 913-929, XP002348797 ISSN: 0968-0896
- PARMAR V S ET AL: "Anti-invasive Activity of Alkaloids and Polyphenolics in Vitro" BIOORGANIC & MEDICINAL CHEMISTRY, ELSEVIER SCIENCE LTD, GB, Bd. 5, Nr. 8, 1997, Seiten 1609-1619, XP002226722 ISSN: 0968-0896
- A. LUPI ET AL.: "Synthetic analogs of natural prenylated and chromene chalcones" FARMACO, EDIZIONE SCIENTIFICA, Bd. 32, Nr. 4, 1977, Seiten 261-269, XP009055305
- V. K. AHLUWALIA ET AL.: "An elegant synthesis of some naturally occuring linear acetylchromenes: eupatoriochromene methyleupatoriochromene (encecalin); evodionol and methylevodionol" TETRAHEDRON, Bd. 38, Nr. 5, 1982, Seiten 609-611, XP002348798
- P. BARUA ET AL.: "Cyclodehydrogenation of ortho-(3,3-dimethylallyl)phenols with trityl tetrafluoroborate" TETRAHEDRON LETT., Bd. 24, Nr. 51, 1983, Seiten 5801-04, XP002348799
- DATABASE CROSSFIRE BEILSTEIN Beilstein Institut zur Förderung der Chemischen Wissenschaften, Frankfurt am Main, DE; XP002348845 Database accession no. BRN 2651050 & BAJWA ET AL.: INDIAN J. CHEM. 11; 100, 1973,
- DATABASE CAPLUS [Online] CHEMICAL ABSTRACTS SERVICE, COLUMBUS, OHIO, US; EL-DAHMY, SAMEEH I.: "Further benzofuran derivatives and the antiinflammatory activity of Helichrysum stoechas (L.) grown in Libya" XP002348846 gefunden im STN Database accession no. 1994:265829 & ZAGAZIG JOURNAL OF PHARMACEUTICAL SCIENCES, Bd. 2, Nr. 1, 1993, Seiten 73-80,
- K. C. NICOLAOU ET AL.: "Natural Product-like Combinatorial Libraries Based on Privileged Structures. 1. General Principles and Solid-Phase Synthesis of Benzopyrans" JOURNAL OF THE AMERICAN CHEMICAL SOCIETY, Bd. 122, Nr. 41, 2000, Seiten 9939-9953, XP002348800
- DATABASE CAPLUS [Online] CHEMICAL ABSTRACTS SERVICE, COLUMBUS, OHIO, US; TSUDA, HIDEYUKI ET AL: "6-Acetyl-8-hydroxy-2,2-dimethylchromene, an antioxidant in sunflower seeds; its isolation and synthesis and antioxidant activity of its derivatives" XP002348847 gefunden im STN Database accession no. 1997:82107 & HETEROCYCLES, Bd. 44, 1997, Seiten 139-142,
- F. BOHLMANN, E. VORWERK: "Synthese natürlich vorkommender p-Hydroxyacetophenon-Derivate, II" CHEM. BER., Bd. 113, 1980, Seiten 261-266, XP009055279
- L. M. HARWOD ET AL.: "An improved procedure for cyclisation of chalcones to flavonones using celite supported potassium fluoride in methanol: total synthesis of bavachinin" SYNTHETIC COMMUNICATIONS, Bd. 20, Nr. 5, 1990, Seiten 649-657, XP009055252
- E. D. BURLING ET AL.: "Studies in the Xanthone Series-VI; Claisen Rearrangement, Selective Demethylationa and the Synthesis of Dihydroisojacareubin and Alvaxanthone Trimethyl Ether" TETRAHEDRON, Bd. 21, 1965, Seiten 2653-2669, XP002348803
- S. J. COUTTS, T. W. WALLACE: "Heterodiene Cycloadditions: Preparation and Transformation of Some Substituted Pyrano[4,3-b][1]benzopyrans" TETRAHEDRON, Bd. 50, Nr. 40, 1994, Seiten 11755-11780, XP002348804
- A. NOHARA ET AL.: "Studies on Antianaphylactic Agents-I; A facile Synthesis of 4-Oxo-4H-1-Benzopyran-3-carbaldehydes by Vilsmeier Reagents" TETRAHEDRON, Bd. 30, 1974, Seiten 3553-3561, XP002348805

## Beschreibung

Die Erfindung bezieht sich auf substituierte Hydroxyacetophenonderivate, die antiproliferative und antimikrobielle Eigenschaften aufweisen, Arzneimittel die diese enthalten, sowie auf ein Verfahren zu deren Herstellung. Darüber hinaus können die erfindungsgemäßen Hydroxyacetophenonderivate als organische Zwischenprodukte für die Herstellung biologisch aktiver Verbindungen dienen.

US 5,449,794 beschriebt die antibakterielle, antivirale oder immunstimmulierende Wirkung von Benzopyranderivaten. Auch WO98/29404 und WO01/60359 beschreiben die Verwendung von Benzopyranderivaten.

Die vorliegende Erfindung stellt substituierte Hydroxyacetophenonderivate, ein Verfahren zu deren Herstellung, sowie auf deren Verwendung in der Herstellung eines antiproliferativen und antimikrobiellen Arzneimittels bereit. Die erfindungsgemäßen Hydroxyacetophenonderivate können darüber hinaus auch als organische Zwischenprodukte für die Herstellung biologisch aktiver Verbindungen verwendet werden.

Die Nachteile der Isolation/Extraktion aus Propolis mit seiner schwankenden und regional unterschiedlichen Zusammensetzung entfallen, auch sind die erfindungsgemäßen Verbindungen bezüglich ihrer Wirkung optimiert.

Die nachstehend genannten Verbindungen sind in der Literatur als Ausgangsstoffe oder Zwischenprodukte bekannt

Die Darstellung von 1-[2-(3-Methylbut-2-enyloxy)phenyl]ethanon aus ortho-Hydroxyacetophenon in Gegenwart von Natriumhydrid als Base in Dimethylsulfoxid ist in Gazz. Chim. Ital. 1989, 119, 385-388 beschrieben. Die elektrochemische Entschützung von 1-[2-(3-Methylbut-2-enyloxy)phenyl]ethanon zu ortho-Hydroxyacetophenon wird in Tetrahedron 2002, 58(45), 9289-9296 beschrieben, und die Titan-katalysierte Entschützung in Tetrahedron Letters 1999, 40(46), 8121-8124.

Die durch Ytterbiumtriflat katalysierte Entschützung von 1-[4-(3-Methylbut-2-enyloxy)phenyl]ethanon zu dem entsprechenden Phenolderivat wird in J. Org. Chem. 1998, 63(24), 9103-9104 beschrieben. In Tetrahedron 2003, 59(27), 5091-5104 wird die säurekatalysierte Synthese von 1-[4-(3-Methylbut-2-enyloxy)phenyl]ethanon ausgehend von 1-[(4-Hydroxy)phenyl]ethanon und 2-Methyl-3-buten-2-ol beschrieben.

In JP 54019948 ist die Synthese von mehreren Chalconderivaten beschrieben. Neben anderen wird auch 1-[2,4-Bis(3-methylbut-2-enyloxy)phenyl]ethanon als Ausgangsmaterial verwendet.

Mitsuo Takasugi, Takahiro Masuda, Phytochemistry, Bd. 43, Nr. 5, 1996, Seiten 1019-1021 offenbart drei 4'-Hydroxyacetophenon-verwandte Phytoalexine aus *Polymnia sonchifolia.*

F. Tomás-Barberán et al., Phytochemistry, Bd. 29, Nr. 4, 1990, Seiten 1093-1095 offenbart antimikrobiell wirkende Phenolverbindungen aus drei spanischen *Helichrysum*-Arten.

Rios, J. L. et al., Journal of Ethnopharmacology, Bd. 33, Nr. 1, 1991, Seiten 51-55 beschreibt die Isolierung und Identifizierung von antibakteriellen Verbindungen aus *Helichrysum stoechas.*

Parmar, V. S. et al., Bioorganic & Medicinal Chemistry, Bd. 11, Nr. 6, 2003, Seiten 913-929 befasst sich mit der Synthese, Charakterisierung und dem in vitro-Screening bzgl. der anti-invasiven Wirkung von polyphenolischen und heterocyclischen Verbindungen.

Auch Parmar, V. S. et al., Bioorganic & Medicinal Chemistry, Bd. 5, Nr. 8, 1997, Seiten 1609-1619 befasst sich mit der anti-invasiven Wirkung von Alkaloiden und Polyphenolen in vitro.

Die substituierten Hydroxyacetophenonderivate der allgemeinen Formel III: 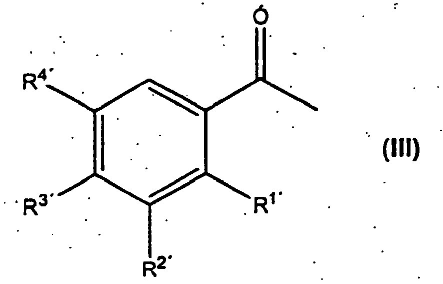 wobei R^{1'}, R^{2'}, R^{3'} und R^{4'} unabhängig voneinander ein Wasserstoffatom, einen Prenylrest, einen 1,1-Dimethylallylrest oder einen 3-Methylbut-2-enyloxyrest darstellen, mit der Maßgabe, dass mindestens ein Rest von R^{1'}, R^{2'}, R^{3'} und R^{4'} ein Wasserstoffatom darstellt und dass mindestens ein Rest von R^{1'}, R^{2'}, R^{3'} und R^{4'} ein 3-Methylbut-2-enyloxyrest ist, können durch Umsetzen eines Hydroxyacetophenonderivats der allgemeinen Formel I: 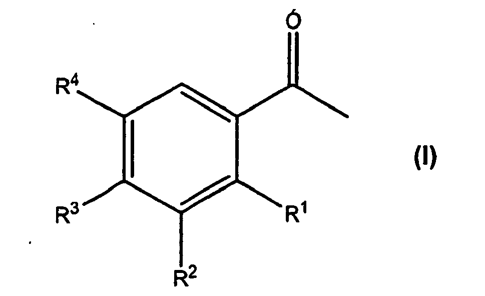 wobei R¹, R², R³ und R⁴ unabhängig voneinander ein Wasserstoffatom, einen Prenylrest, einen 1,1-Dimethylallylrest oder eine Hydroxygruppe darstellen, mit der Maßgabe, dass mindestens ein Rest von R¹, R², R³ und R⁴ ein Wasserstoffatom darstellt und dass mindestens ein Rest von R¹, R², R³ und R⁴ eine Hydroxygruppe ist, in Gegenwart einer Base, bei einer Reaktionstemperatur von 10 bis 50 °C, in einem organischen Lösungsmittel mit einem 3-Methylbut-2-enylhalogenid der Formel II: 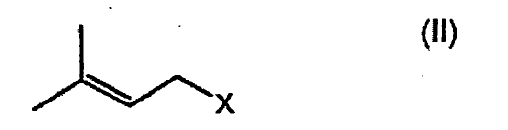 wobei X ein Chlor-, Brom- oder Iodatom darstellt, hergestellt werden [O-Allylierung gemäß der allgemeinen Arbeitsvorschrift 1 (AAV1), siehe auch nachstehendes Formelschema (1)].

Vorzugsweise werden nach AAV1 Verbindungen der allgemeinen Formel III dargestellt, in der R^{1'}und / oder R^{3'} einen 3-Methylbut-2-enyloxyrest darstellt, d.h. sie werden aus Verbindungen der allgemeinen Formel I dargestellt, in der R¹ und / oder R³ eine Hydroxygruppe darstellt.

Die O-Allylierung wird vorzugsweise in Dimethylformamid (DMF) als Lösungsmittel, bei einer Reaktionstemperatur von 20 bis 40 °C in Gegenwart der Base Kaliumcarbonat durchgeführt (siehe AAV1).

Die so hergestellten, O-allylierten Verbindungen der allgemeinen Formel III können anschließend nach den allgemeinen Arbeitsvorschriften 2 oder 3 (AAV2 oder AAV3) in N,N-Diethylanilin und bei einer Reaktionstemperatur von 160 bis 220 °C (vorzugsweise unter Rückfluss) zu den entsprechenden C-prenylierten bzw. C-alkylierten Verbindungen der allgemeinen Formel IV: 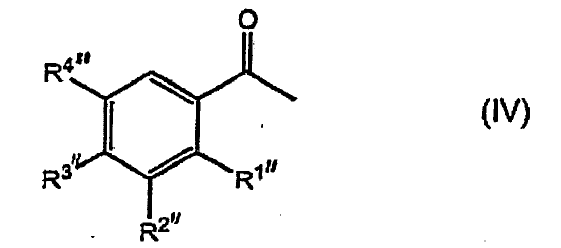 wobei R^{1"}, R^{2"}, R^{3"} und R^{4"} unabhängig voneinander ein Wasserstoffatom, einen Prenylrest, einen 1,1-Dimethylallylrest oder eine Hydroxygruppe darstellen, mit der Maßgabe, dass mindestens ein Rest von R^{1"}, R^{2"}, R^{3"} und R^{4"} ein Prenylrest oder ein 1,1-Dimethylallylrest ist und dass mindestens ein Rest von R^{1"}, R^{2"}, R^{3"} und R^{4"} eine Hydroxygruppe ist, umgesetzt werden [siehe nachstehendes Formelschema (2)].

Vorzugsweise werden nach AAV2 oder AAV3 Verbindungen der allgemeinen Formel IV dargestellt, in der R^{1"} und/oder R^{3"} eine Hydroxygruppe darstellt.

Die Verbindungen der allgemeinen Formel IV, in der R^{1"} für einen Hydroxyrest steht, können des weiteren mit mindestens 2 Moläquivalenten Phosphoroxychlorid (POCl₃) in DMF bei einer Reaktionstemperatur von 40 bis 50 °C (vorzugsweise 45 °C) zu den entsprechenden 4-Oxo-4H-chromen-3-carbaldehydderivaten der allgemeinen Formel V, wobei R^{2"}, R^{3"} und R^{4"} unabhängig voneinander ein Wasserstoffatom, einen Prenylrest, einen 1,1-Dimethylallylrest oder eine Hydroxygruppe darstellen, mit der Maßgabe, dass mindestens ein Rest von R^{2"}, R^{3"} und R^{4"} ein Prenylrest oder ein 1,1-Dimethylallylrest ist, umgesetzt werden [siehe nachstehendes Formelschema (3)].

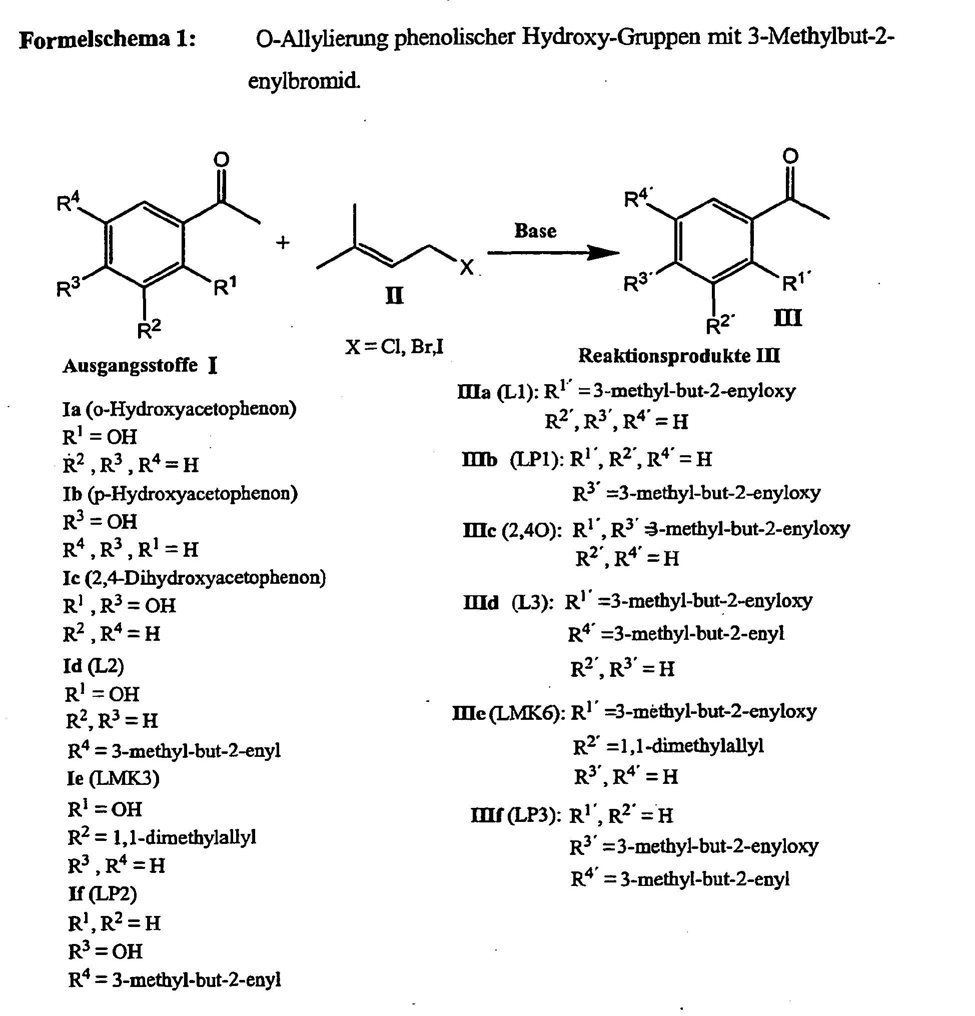

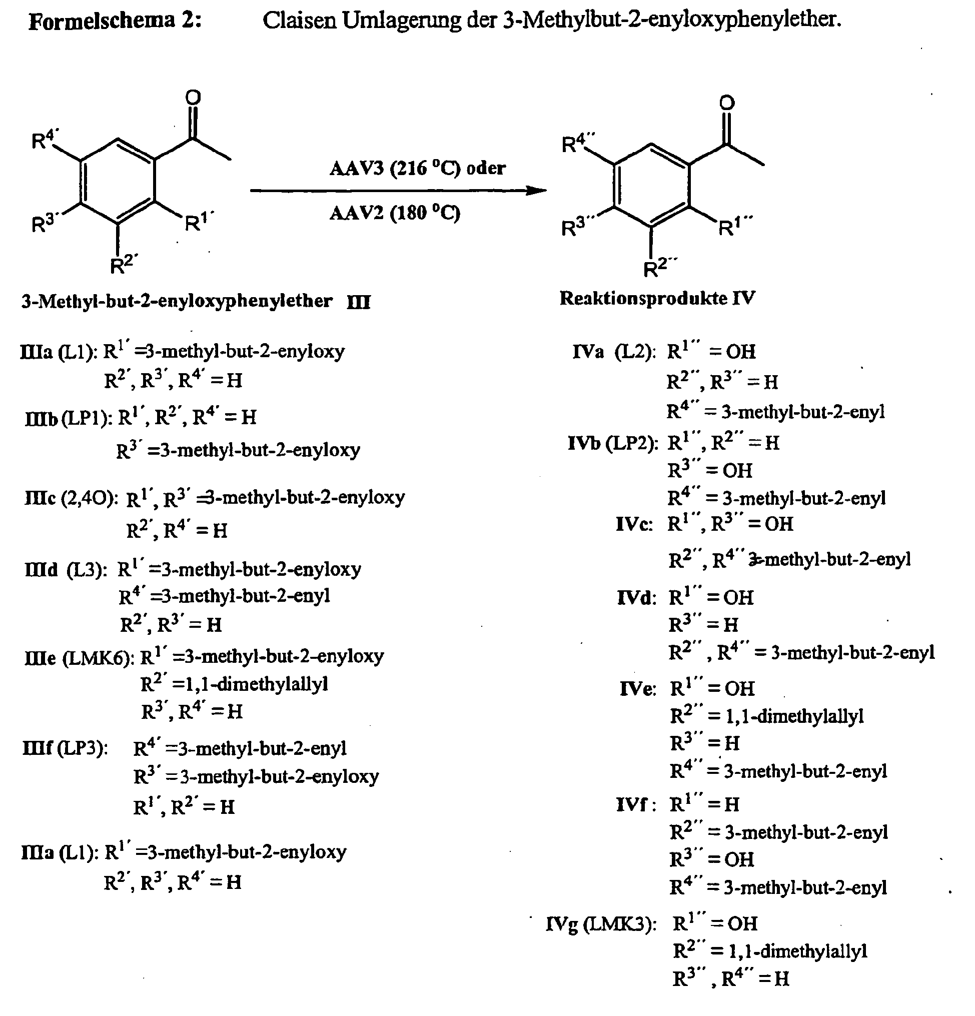

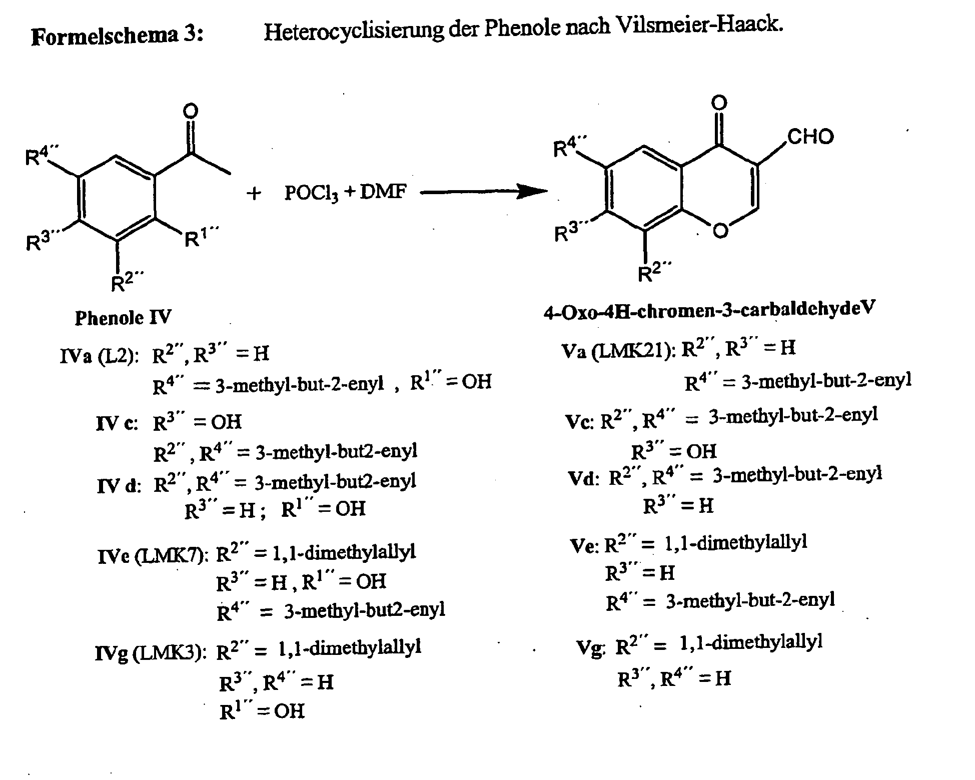

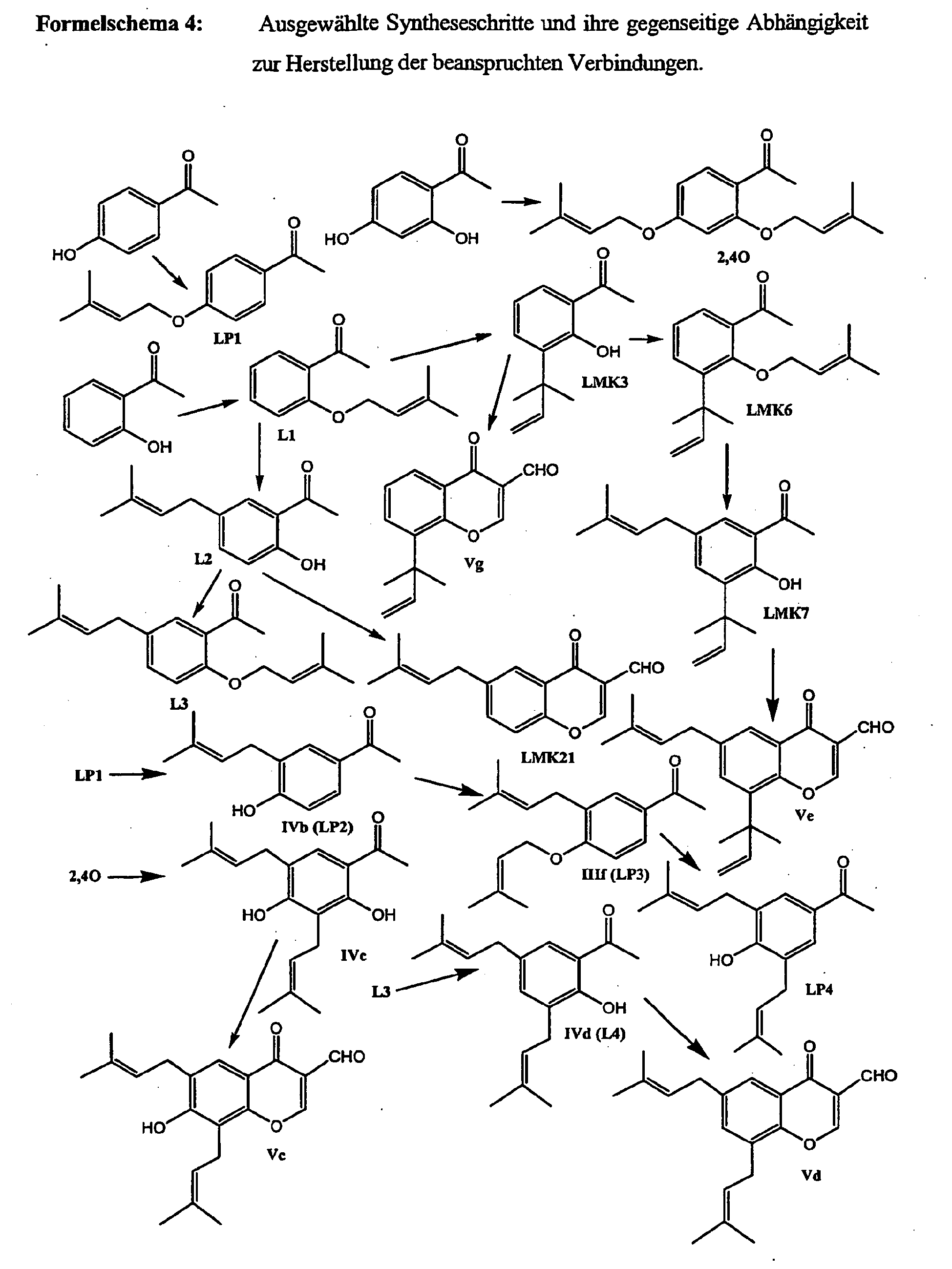

Sowohl die nach den vorstehend genannten Verfahren hergestellten erfindungsgemäßen Hydroxyacetophenonderivate der Formeln III und IV (nicht im umfang der Erfindung enthalten) als auch die 4-Oxo-4H-chromen-3-carbaldehydderivate der Formel V (nicht im umfang der Erfindung enthalten) zeigen antiproliferative und/oder antimikrobielle Eigenschaften und können somit erfindungsgemäß zur Herstellung von antiproliferativen oder antimikrobiellen Arzneimitteln verwendet werden.

Die Hydroxyacetophenonderivate der Formeln III wurden, mit Aufnahme von 1-[2-(3-Methylbut-2-enyloxy)phenyl]ethanon, 1-[4-(3-Methylbut-2-enyloxy)phenyl]ethanon und 1-[2,4-Bis(3-methylbut-2-enyloxy)phenyl]ethanon, noch nicht beschrieben und stellen somit einen weiteren Aspekt der vorliegenden Erfindung dar.

Bevorzugte Verbindungen der vorliegenden Erfindung sind die nachstehende Verbindungen der Formel III:
1-[5-(3-Methylbut-2-enyl)-2-(3-methylbut-2-enyloxy)phenyl]ethanon,
1-[3-(1,1-Dimethylallyl)-2-(3-methylbut-2-enyloxy)phenyl]ethanon und
1-[5-(3-Methylbut-2-enyl)-4-(3-methylbut-2-enyloxy)phenyl]ethanon:

### Allgemeine Arbeitsvorschriften (AAV) zur Synthese von Hydroxyacetophenonderivaten

### AAV1: O-Allylierung phenolischer Hydroxy-Gruppen mit 3-Methylbut-2-enylbromid

Zu einer gerührten Lösung von 2 mmol des entsprechenden Hydroxyacetophenons der allgemeinen Formel I in 10 ml Dimethylformamid werden 6 mmol Kaliumcarbonat gegeben. Die Lösung wird bei Raumtemperatur 30 Minuten unter Argonatmosphäre gerührt. Anschließend werden 3 mmol 3-Methylbut-2-enylbromid in die Lösung unter fortlaufendem Rühren injiziert. Nach ca. 8-stündigem fortlaufenden Rühren unter Schutzgas bei 40° C wird die Mischung in 20 ml eines Eis-Wasser-Gemisches gegossen. Die wässrige Suspension wird 3mal mit jeweils 30 ml Chloroform extrahiert. Anschließend wird die organische Phase mit einer Natriumhydrogensulfat-Lösung und darauf folgend mit Wasser gewaschen. Die Trocknung der Chloroformphase erfolgt über Natriumsulfat. Nach Filtration wird die Lösung unter vermindertem Druck eingeengt und der Rückstand chromatographisch aufgearbeitet.

### AAV2: CLAISEN-Umlagerung der 3-Methylbut 2-enyloxyphenylether

1 mmol des entsprechenden Allylphenylethers der allgemeinen Formel III wird in 10 ml N,N-Diethylanilin gelöst. Die Lösung wird 5 Stunden auf 180°C erhitzt. Nach dem Abkühlen der Reaktionslösung wird diese in 50 ml Chloroform aufgenommen. Die Chloroform-Lösung wird 3mal mit 15%iger Salzsäure und anschließend mit Wasser neutral gewaschen. Danach wird das Chloroform unter vermindertem Druck destillativ entfernt und der Rückstand in 20 ml Methanol aufgenommen. Zu dieser Lösung wird eine Spatelspitze Amberlit IR-120 zugegeben und diese Mischung 20 Minuten gerührt. Nach Filtration der Lösung wird diese unter vermindertem Druck eingeengt und der Rückstand chromatographisch aufgearbeitet.

### AAV3: CLAISEN-COPE-Umlagerung der 3-Methylbut-2-enyloxyphenylether

1 mmol des entsprechenden 3-Methylbut-2-enyloxyphenylethers der allgemeinen Formel III wird in 10 ml N,N-Diethylanilin aufgenommen. Diese Lösung wird anschließend 8 Stunden unter Rückfluss erhitzt (216°C). Nach Ablauf dieser Reaktionszeit wird das Gemisch in 50 ml Chloroform gelöst. Das N,N-Diethylanilin wird mit 15%iger Salzsäure ausgeschüttelt und die zurückbleibende organische Phase mit Wasser neutral gewaschen. Die Chloroformphase wird abgetrennt und das Chloroform unter vermindertem Druck destillativ entfernt. Der Rückstand wird in 20 ml Methanol gelöst und nach Zugabe einer Spatelspitze Amberlit IR-120 20 Minuten gerührt. Die Lösung wird filtriert und unter vermindertem Druck eingeengt. Der Rückstand wird chromatographisch aufgearbeitet

### Ausführungsbeispiele

### 1. Synthese von 1-[2-(3-Methylbut-2-enyloxy)phenyl]ethanon (L1)

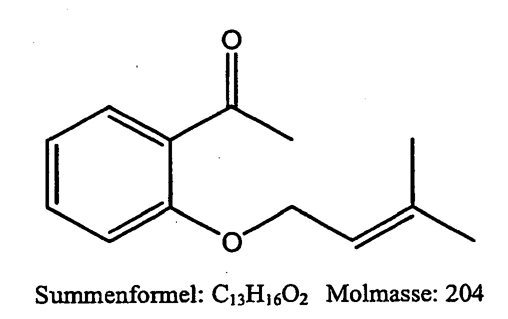

1-(2-Hydroxyphenyl)ethanon wird mit 3-Methylbut-2-enylbromid entsprechend AAV 1 umgesetzt. Bei der chromatographischen Aufarbeitung wurde als Eluent eine Mischung bestehend aus Toluen / Ethylacetat = 9 / 1 verwendet.

Ausbeute: 85 % als farblose Flüssigkeit; gemessener Rf-Wert (DC, Toluol:Ethylacetat 9:1): 0,41

### Strukturelle Identifizierung der synthetisierten Verbindung L1 durch Kernresonanz (NMR)- und Infrarotspektroskopie (IR):

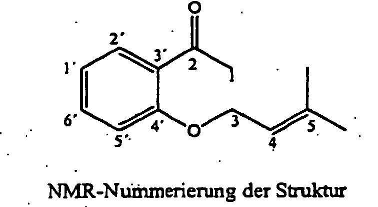

**¹H-NMR (DMSO-d₆**): 7,6 (d, 2H, H-1'/H-5'), 7,1 (d, 2H, H-2'/H-6'), 5,44 (m, 1H, H-4), 4,6 (d, 2H, H-3), 2,6 (s, 3H, CH₃CO), 1,8 (s, 3H, CH₃ prenyl), 1,7(s,3H, CH₃ prenyl).
**¹³C-NMR (CDCl₃):** 200,1 (CO), 158,3 (C-4'), 138,3 (C-5), 133,5 (C-6'), 130,4 (C-2'), 128,5 (C-3'), 120,4 (C-1'), 119,1 (C-4), 112,7 (C-5'), 65,3 (C-3), 32,0 (C-1), 25,7 (CH₃), 18,2 (CH₃).
**IR (aufgenommen als Film**): 3072, 3028 (=CH), 2976, (CH₃), 2879 (CH₂),1674 (C=0), 1236 (C-O) cm⁻¹.

### 2. Synthese von 1-[2-Hydroxy-5-(3-methylbut-2-enyl)phenyl]ethanon (L2) Referenzbeispiel

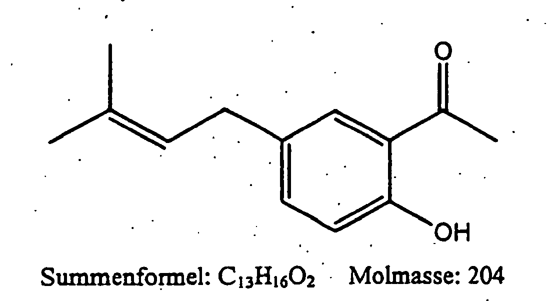

Das unter Ausführungsbeispiel 1 erhaltene 1-[2-(3-Methylbut-2-enyloxy)phenyl]ethanon (L1) wird entsprechend AAV 3 umgesetzt. Bei der Aufarbeitung wurde als Eluent eine Mischung bestehend aus Toluen / Ethylacetat = 9,5 / 0,5 verwendet.
Ausbeute: 70 % als farblose Flüssigkeit; gemessener Rf-Wert (DC, Toluol: Ethylacetat. 9:1): 0,79

### Strukturelle Identifizierung der synthetisierten Verbindung L2 durch Kernresonanz (NMR) -, Infrarot (IR) - und Massenspektrometrie (MS):

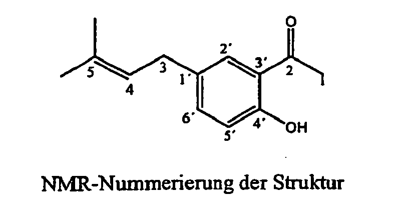

**¹H-NMR (DMSO-d₆)**: 12,1 (s, 1H, OH), 7,5 (d, 1H, H-6'), 7,2 (d, 2H, H-2'/H-5'), 5,3 (m, 1H, H-4), 3,3 (d, 2H, H-3), 2,5 (s, 3H, H-1), 1,8 (s, 3H, CH₃ prenyl), 1,7 (s, 3H, CH₃ prenyl)
**¹³C-NMR (CDCl₃)**: 204,4 (CO), 160,5 (C-4'), 136,8 (C-6'), 133,1 (C-5), 132,1 (C-1'), 129,6(C-2'), 122,7(C-4), 119,3(C-3'), 118,2 (C-5'), 33,3(C-3), 26,6 (CH₃CO), 25,7 (CH₃ prenyl), 17,8 (CH₃ prenyl)
**IR (aufgenommen als Film**): 3437 (OH), 3029 (HC= aromat), 2971, 2916 (CH₃), 2857, (CH₂), 1642 (C=O), 1589 (C=C) cm⁻¹.
**MS (70 eV**): 204 M^{+.} ; 189 (M^{+.}-15(CH₃)); 161 (M^{+.} -43 (CH₃CO); 69 (C₅H₉, prenyl); 43 (CH₃CO).

### 3. Synthese von 1-[3-(1,1-Dimethylallyl)-2-hydroxyphenyl]ethanon (LMK 3) Referenzbeispiel

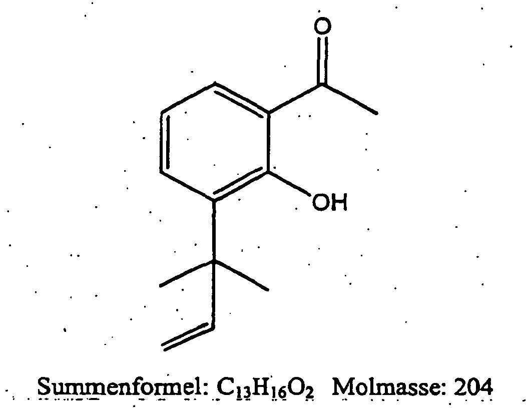

Das unter Ausführungsbeispiel 1 erhaltene 1-[2-(3-Methylbut-2-enyloxy)phenyl]ethanon (L1) wird entsprechend AAV 2 umgesetzt. Bei der Aufarbeitung wurde als Eluent eine Mischung bestehend aus Toluen / Ethylacetat = 9,5 / 0,5 verwendet.

Ausbeute: 28 % als farblose Flüssigkeit; gemessener Rf-Wert (DC, Toluol:Ethylacetat 9,5:0,5): 0,71

### Strukturelle Identifizierung der synthetisierten Verbindung LMK3 durch Kernresonanz (NMR) -, Infrarot (IR) - und Massenspektrometrie (MS):

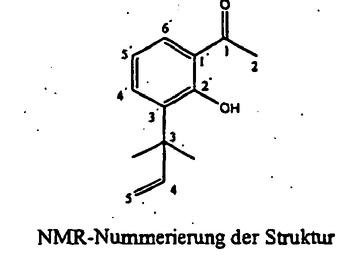

**¹H-NMR (CDCl₃)**: 13.1 (s, 1H, OH); 7.64 (dd, 1H, H-6'); 7.49 (dd, 1H, H-4'); 6.83 (m, 1H, H-5'); 6.25 (dd, 1H, H-4); 5.29 (dd, 1H, vinyl CH₂); 4.97 (dd, 1H, vinyl CH₂); 2.63 (s, 3H, CH₃CO); 1.5 (s, 6H, CH₃-allyl).
**¹³C-NMR (75 MHz, CDCl₃**): 205.1 (CO); 161.7 (C-2'); 147.2 (C-4, vinyl-CH); 134.1 (C-3'); 132.1 (C-3'); 129.1 (C-6'), 124.3 (C-1'); 119.6 (C-5'); 110.5 (C-5, vinyl-CH₂); 40.6 (C-3); 27.0(C-2); 26.8 ((CH₃)₂C-5).
**IR (aufgenommen als Film**): 3435 (OH), 3030 (=CH aromat), 2970, 2915 (CH₃), 2855, (CH₂), 1644 (C=O), 1587 (C=C) cm⁻¹.
MS (70 eV): 204 M^{+.} ; 189 (M^{+.} -15(CH₃)); 161 (M^{+.} -43 (CH₃CO); 43 (CH₃CO).

### 4. Synthese von 1-[3-(1,1-Dimethyl-allyl)-2-(3-methyl-but-2-enyloxy)-phenyl]-ethanon (LMK6)

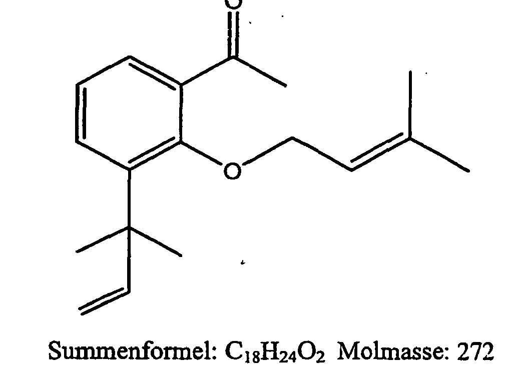

Das unter Ausführungsbeispiel 3 erhaltene 1-[3-(1,1-Dimetylallyl)-2-hydroxyphenyl]-ethanon (LMK3) wird mit 3-Methylbut-2-enylbromid entsprechend AAV 1 umgesetzt. Bei der chromatographischen Aufarbeitung wurde als Eluent eine Mischung bestehend aus Toluen / Ethylacetat = 9,5 / 0,5 verwendet.
Ausbeute: 67% als farblose Flüssigkeit; gemessener Rf-Wert (DC, Toluol:Ethylacetat 9:1)
: 0,37

### Strukturelle Identifizierung der synthetisierten Verbindung LMK6 durch Kernresonanz (NMR) -, Infrarot (IR) - und Massenspektrometrie (MS):

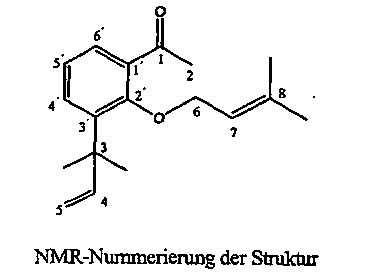

**¹H-NMR (CDCl₃):** 7.43 (dd, 1H, H-6'); 7.33 (dd, 1H, H-4'); 7.07 (m, 1H, H-5'); 6.21 (dd, 1H, H-4); 5.48 (m, 1H, H-7); 5.03 (dd, 1H, H-5, vinyl CH₂); 4.98 (dd, 1H, H-5, vinyl-CH₂); 4.27 (d, 2H, H-6, O-CH₂); 2.61 (s, 3H, CH₃CO); 1.79 (s, 3H, CH₃-prenyl); 1.63 (s, 3H, CH₃- prenyl); 1.51 (s, 6H, (CH₃)₂C-3).
**¹³C-NMR (75 MHz, CDCl₃)**: 203.0 (CO); 157.0 (C-2'); 148.3 (C-4, vinyl-CH); 136.8 (C-8); 131.3 (C-3'); 128.2 (C-8), 127.6 (C-4'); 123.2 (C-6'); 120.3 (C-1'); 120.1 (C-5'); 120.0 (C-7); 110.3 (C-5); 40.7 (C-3); 29.9 (C-2); 28.2 ((CH₃)₂C-3); 25.7 (CH₃-prenyl) ; 18.3 (CH₃-prenyl).
**IR (aufgenommen als Film)**: 3032 (=CH aromat), 2975, 2917 (CH₃), 2850, (CH₂), 1675 (C=O), 1585 (C=C) cm⁻¹.
**MS (70 eV)**: 272 M^{+.} ; 257 (M^{+.}- 15(CH₃)); 229 (M^{+.} -43 (CH₃CO); 43 (CH₃CO).

### 5. Synthese von 1-[3-(1,1-Dimetylallyl)-2-hydroxy-5-(3-methylbut-2-enyl)phenyl]-ethanon (LMK7) Referenzbeispiel

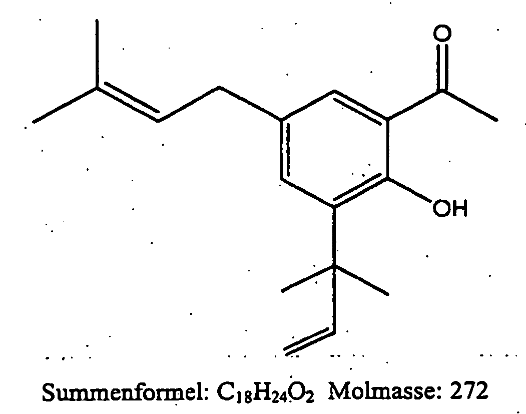

Das unter Ausführungsbeispiel 4 erhaltene 1-[3-(1,1-Dimethylallyl)-2-(3-methylbut-2-enyloxy)phenyl]ethanon (LMK6) wird entsprechend AAV 3 umgesetzt. Bei der chromatographischen Aufarbeitung wurden als Eluent eine Mischung bestehend aus Toluen / Ethylacetat = 9,5 / 0,5 verwendet.
Ausbeute: 23 % als farblose Flüssigkeit

### Strukturelle Identifizierung der synthetisierten Verbindung LMK7 durch Kernresonanz (NMR) -, Infrarot (IR) - und Massenspektrometrie (MS):

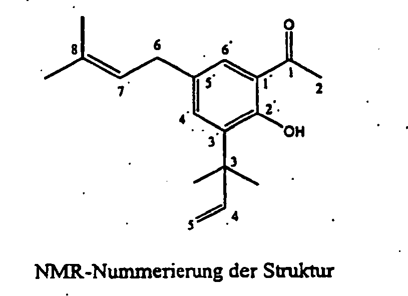

**¹H-NMR (CDCl₃)**: 12.92 (s, 1H, OH); 7.40 (d, 1H, -H-6'); 7.31 (d, 1H, H-4'); 6.25 (dd, 1H, H-4); 5.30 (m, 1H, H-7); 5.02 (dd, 1H, H-5,vinyl-CH₂); 4.97 (dd, 1H, H-5, vinyl-CH₂); 3.28 (d, 2H, H-6); 2.61 (s, 3H, CH₃CO); 1.77 (s, 3H, CH₃-prenyl); 1.73 (s, 3H, CH₃- prenyl); 1.50 (s, 6H, (CH₃)₂C-3).
**¹³C-NMR (75 MHz, CDCl₃**): 204.5 (CO); 160.2 (C-2'); 147.6 (C-4, vinyl-CH); 136.8 (C-8); 134.7 (C-3'); 131.1 (C-4'); 129.9 (C-5'); 123.2 (C-6'); 117.8 (C-1'); 110.1 (C-5); 40.6 (C-3); 38.1 (C-6); 34.1 (CH₃CO); 33.9 ((CH₃)₂C-3); 26.2 (CH₃-prenyl); 18.1 (CH₃-prenyl.
**IR (aufgenommen als Film**): 3035 (=CH aromat), 2973, 2920 (CH₃), 2847, (CH₂), 1643 (C=O), 1580 (C=C) cm⁻¹.
MS (70 eV): 272 M^{+.} ; 257 (M^{+.} -15(CH₃)); 229 (M^{+.} -43 (CH₃CO); 43 (CH₃CO).

### 6. Synthese von 1-[5-(3-Methylbut-2-enyl)-2-(3-methylbut-2-enyloxy)phenyl]-ethanon (L3)

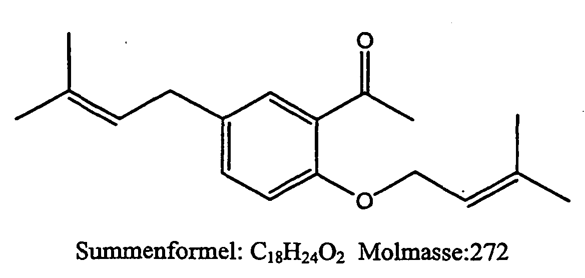

Das unter Ausführungsbeispiel 2 erhaltene 1-[2-Hydroxy-5-(3-methylbut-2-enyl)phenyl]-ethanon (L2) wird mit 3-Methylbut-2-enylbromid entsprechend AAV 1 umgesetzt. Bei der chromatographischen Aufarbeitung wurde als Eluent eine Mischung bestehend aus Toluen / Ethylacetat = 9 / 1 verwendet.
Ausbeute: 80 %

### Strukturelle Identifizierung der synthetisierten Verbindung L3 durch Kernresonanz (NMR)- und Infrarotspektroskopie (IR):

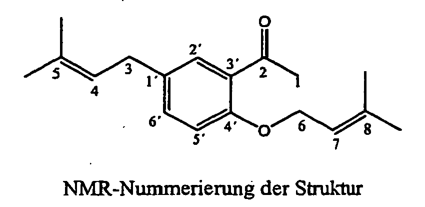

**¹H-NMR (DMSO-d₆**): 7,5 (d, 1H, H-2'), 7,2 (d, 1H, H-6'), 6,8 (d, 1H, H-5'), 5,5 (m, 1H, H-4), 5,2 (m, 1H, H-7), 4,6 (d, 2H, H-6), 3,2 (d, 2H, H-3), 2,5 (s, 3H, H-1), 1,8 (s, 6H, CH₃ prenyl), 1,7 (s, 6H, CH₃ prenyl).
**IR (aufgenommen als Film):** 3070, 3026 (=CH), 2970 (CH₃), 2879 (CH₂), 1678 (C=O), 1236(C-O) cm⁻¹.

### 7. Synthese von 1-[4-(3-Methylbut-2-enyloxy)phenyl]ethanon (LP1)

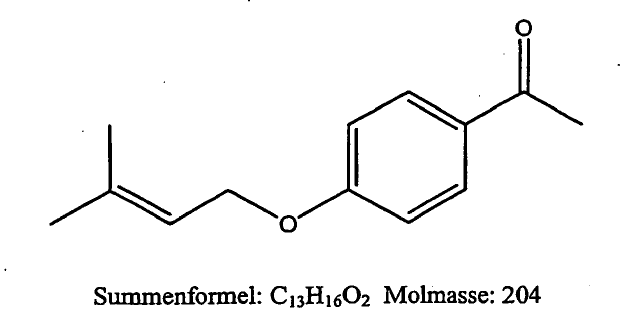

1-(4-Hydroxyphenyl)ethanon wird mit 3-Methylbut-2-enylbromid entsprechend AAV 1 umgesetzt. Bei der Aufarbeitung wurde das als fester Stoff ausgefallene 1-[4-(3-Methylbut-2-enyloxy)phenyl]ethanon (LP1) mit Wasser gewaschen und bei Raumtemperatur getrocknet.
Ausbeute: 80 % als farblose Verbindung; Fp: 47 °C

### Quantitative Identifizierung der synthetisierten Verbindung LP1 durch Elementaranalyse:

Berechnete Massenrelationen lt. Formelzusammensetzung: 76,47% C; 7,84% H
Gefundene Massenrelationen bestätigen die Summenformel: 76,43% C; 7,81 % H

### Strukturelle Identifizierung der synthetisierten Verbindung LP1 durch Kernresonanz (NMR) -, Infrarot (IR) - und Massenspektrometrie (MS):

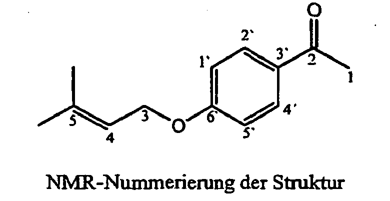

**¹H-NMR (DMSO-d₆)**: 7,9 (d, 2H, H-2'/H-4'), 6,9 (d, 2H, H-1'/H-5'), 5,5 (m, 1H, H-4), 4,6 (d, 2H, H-3), 2,6 (s, 3H, CH₃CO), 1,8 (s, 3H, CH₃ prenyl), 1,7 (s, 3H, CH₃ prenyl) ¹³C-NMR (CDCl₃): 196,7 (CO), 162, 7(C-6'), 138,8 (C-5), 130,5 (C-2';C-4'), 130,1 (C-3'), 118,8 (C-4), 114,2 (C-5'; C-1'), 64, 9 (C-3), 26, 2 (C-1), 25,7 (CH₃), 18,1 (CH₃).
**IR (aufgenommen als Film**): 3072, 3029 (=CH), 2970, 2935 (CH₃), 2875 (CH₂), 1668 (C=0), 1242 (C-O) cm⁻¹.
**MS (70eV**): 204 M +, 136 (M +- 68), 69 (prenyl).

### 8. Synthese von 1-[2,4-Bis(3-methylbut-2-enyloxy)phenyl]ethanon (2,40)

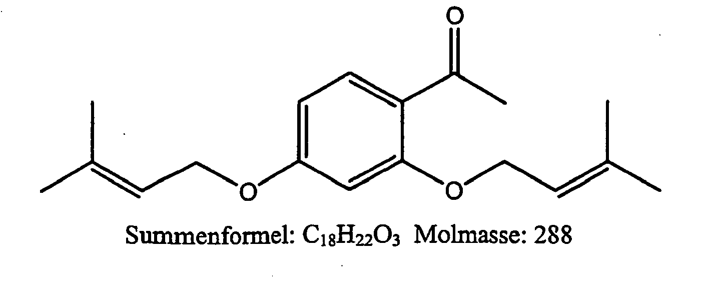

1-(2,4-Dihydroxyphenyl)ethanon wird mit 3-Methylbut-2-enylbromid entsprechend AAV 1 umgesetzt. Bei der chromatographischen Aufarbeitung wurde als Eluent eine Mischung, bestehend aus Toluen / Ethylacetat = 9 /1 verwendet.

### Strukturelle Identifizierung der synthetisierten Verbindung 2,40 durch Kernresonanz (NMR) - Spektrometrie:

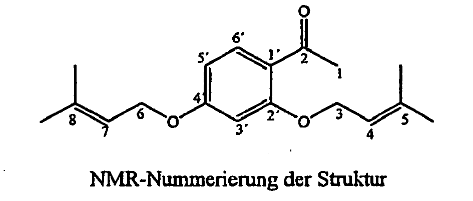

**¹H-NMR (DMSO-d₆)**: 7,6 (d, 1H, H-6'), 6,5 (d, 1H, H-5'), 6,4 (d, 1H, H-3'), 5,6 (m, 1H, H-7), 5,5 (m, 1H, H-4), 4,7 (d, 2H, H-6), 4,6 (d, 2H, H-3), 2,5 (s, 3H, H-1), 1,8 (s, 6H, CH₃ prenyl), 1,7 (s, 6H, CH₃ prenyl).

### 9. Synthese von 6-(3-Methylbut-2-enyl)-4-oxo-4H-chromen-3-carbaldehyd (LMK21) Referenzbeispiel

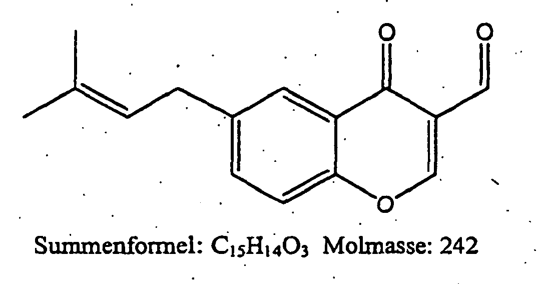

4 mmol des unter Ausführungsbeispiel 2 erhaltenen 1-[2-Hydroxy-5-(3-methylbut-2-enyl)-phenyl]ethanon (L2) werden in 2 mL wasserfreiem Dimethylformamid gelöst. Unter Kühlung fügt man tropfenweise 1,29g Phosphoroxychlorid hinzu. Die Reaktionsmischung wird unter Rühren 1 Stunde bei 45 °C erhitzt. Anschließend wird die Reaktionsmischung auf Eiswasser gegossen und danach 4 Stunden beim Raumtemperatur gerührt. Die wässrige Phase wird mit Chloroform dreimal extrahiert, über Natriumsulfat getrocknet, im Vakuuni eingeengt und säulenchromatographisch gereinigt (n-Hexan / Ethylacetat =1/0,1)
Ausbeute: 11 %

### Strukturelle Identifizierung der synthetisierten Verbindung LMK21 durch Kernresonanz (NMR) -, Infrarot (IR) - und Massenspektrometrie (MS):

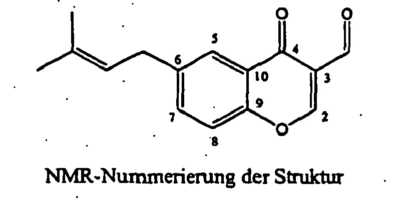

**¹H-NMR (CDCl₃)**: 10.35 (s, 1H, CHO); 8.52 (s, 1H, H-2); 8.07 (d, 1H, H-5); 7.56 (m, 1H, H-7); 7.44 (d, 1H, H-8); 5.32 (m, 1H, =CH vinyl); 3.46 (d, 2H, CH₂); 1.77 (s, 3H, CH₃-prenyl); 1.73 (s, 3H, CH₃-prenyl).
**¹³C-NMR (75 MHz CDCl₃**): 188.6 (CHO); 175.3 (C-4); 164.1 (C-2); 156.1 (C-9); 135.7 (C-7), 127.2 (C(CH₃)₂), 125.8(C-6); 125.1 (C-10); 123.6 (C-5); 119.2 (C-8); 117.9 (=CH-prenyl); 36.5 (CH₂-prenyl); 25.1 (CH₃-prenyl); 18.9 (CH₃-prenyl).
**IR (aufgenommen als Film**): 3040, 3020 (=C-H), 2975 (CH₃), 2870 (CH₂), 1695 (CHO) e 1645 (CO) cm⁻¹.
MS (70 eV): 242 M^{+.}, 241 (M^{+.} -1 (H), 69 (prenyl), 29 (CHO).

### Antitumorale Wirkung der erfindungsgemäßen Verbindungen

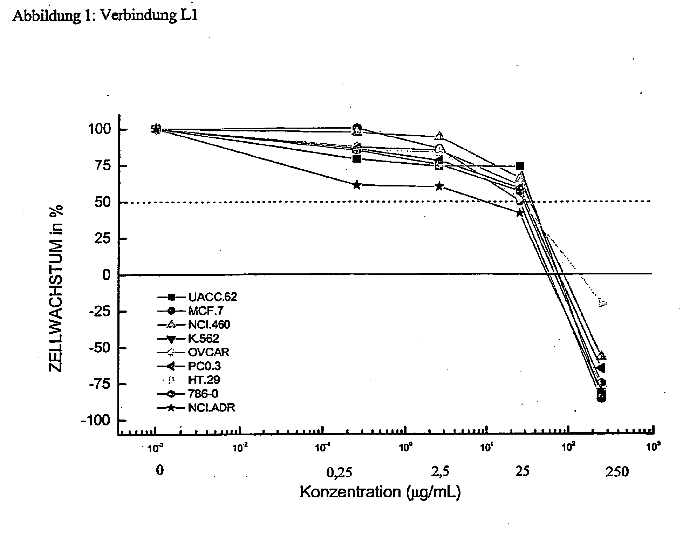

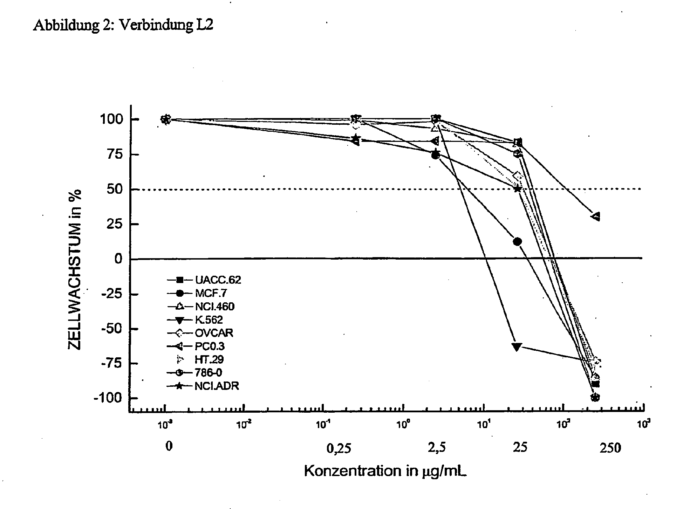

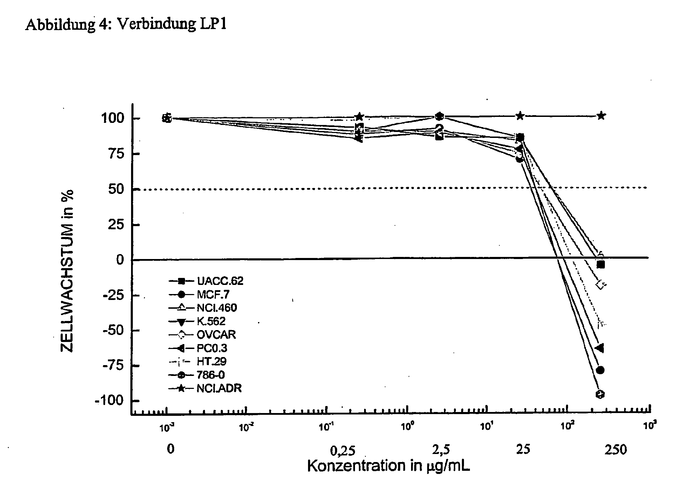

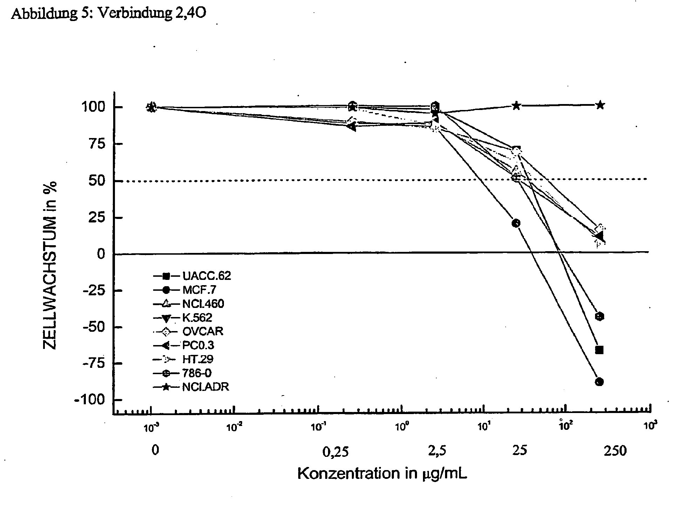

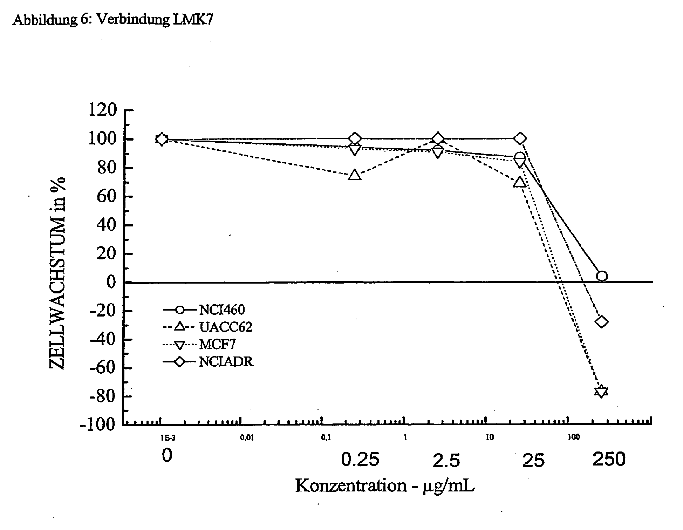

### (Testergebnisse zur Antitumor-Aktivität der synthetisierten Verbindungen (siehe Abbildungen 1-6))

Die Tests wurden mit folgenden humanen Krebszell-Linien durchgeführt:
Lungen NCI-460, Mama (Breast) Normal MCF-7, Mama NCI-ADR (exprimiert MDR-Phänotyp), Hautkrebs (Melanoma) UACC-62, Leukemia K-562 Colon HT-29, Nierenkrebs 786-0, Ovarienkrebs OVCAR-3 und Prostatakrebs PC-3 (ein Geschenk des National Cancer Institute, Frederik, MA, USA).
Die Zellen wurden in 25ml Zelkulturflaschen (Nunc) mit 5ml RPMI 1640 (Gibco BRL, Life Technologies) mit 5% fötalem Kälberserum kultiviert.
Die adhärierenden Zellen wurden trypsiniert (0,5 ml Trypsin, Nuticell Nutrientes Cellulares). Nachfolgend wurde das Trypsin durch Zugabe von 5ml 5% Serum in RPMI 1640 inaktiviert. Es wurde eine Einzelzellsuspension durch behutsames Pipettieren hergestellt, die Zellen wurden gezählt, Verdünnungen mit passender Zelldichte, die nach Zell-Linie varierte, hergestellt und 100µl/Vertiefung wurde in Mikrotiterplaten mit 96 Vertiefungen ausgesäät.
Die Mikrotiterplatten wurde für 24 Stunden bei 37°C zur Stabilisierung der Kulturen vorinkubiert. Die Zellen wurden mit 100 µl Verdünnungen der Testsubstanzen (0,25; 2,5; 25 und 250 µg/ml, in dreifacher Ausführung) bei 37°C und 5% CO₂ 48 Stunden inkubiert.
Als positive Kontrollen dienten Doxirubicin (Sigma Chemical Co.) und Tamoxifen (Sigma Chemical Co.).
Der Sulforhodamin B (SRB) Test auf die antiproliferative Wirkung wurde gemäß der Vorschriften von Skehan et al. (Journal of National Cancer Insitute Vol.82, pp. 1107-1118, 1990*)* durchgeführt. Die Zellen wurden mittels Proteinfällung mit 50% Trichloressigsäure (TCA, Sigma Chemicals Co.) (50 µl/Vertiefung, Endkonzentration 10%)) bei 4°C für eine Stunde fixiert. Der Überstand wurde verworfen und die Platten 5 mal mit Leitungswasser gewaschen. Die Zellen wurden für 30 Minuten mit 0,4% SRB in 1% Essigsäure (50 µl/Vertiefung) gefärbt und anschliessend 4 mal mit 1% Essigsäure gewaschen um den nichtgebundenen Farbstoff zu entfernen. Die Platten wurden getrocknet und der gebundene Farbstoff mit 150 µl/Vertiefung 10mM Trizma-Puffer (Sigma Chemicals Co.) solubilisiert. Die optische Dichte wurde mit einem automatisierten Plattenlesegerät (Molecular Devices Max Microplate Reader) bei 540 nm bestimmt. Alle Bestimmungen wurden als Dreifachbestimmung durchgeführt. Die optische Dichte wurde mit dem Excel® Programm (Microsoft Office Package) errechnet.

Die erfindungsgemässen Verbindungen zeigen sowohl cytostatische als auch cytocide Aktivität bei Konzentrationen zwischen 0.5 bis 250 µg/ml. (1-[2-Hydroxy-5-(3-methylbut-2-enyl)ethanon Referenzbeispiel zeigte selektive Eigenschaften, ohne die Fibroblasten zu schädigen und war bei einer Verdünnung von 1:2000 noch aktiv.

Die Hemmung des Zellwachstums in Abhängigkeit von der Konzentration ausgewählter erfindungsgemäßer Verbindungen wird in den Abbildungen 1 bis 6 gezeigt.

### Abbildung 1

Die Verbindung L1 zeigte cytostatische Aktivität (Inhibition des Zellenwachsturns) gegen alle getesteten Zell-Linien und cytocide Aktivität gegen NCI-460, UACC-62 und NCl-ADR.

### Abbildung 2

Die Verbindung L2 Referenzbeispiel zeigte eine sehr starke Wirkung gegen alle Zell-Linien bei Konzentrationen zwischen 0,5 und 250 µg/mL.

### Außerdem wurde diese Substanz an folgenden Zell-Linien getestet:

MIAPaCa2 pancreatic; C205 colon und T47D breast und als Vergleich an der nicht pathogenen Linie: WI38 (Fibroblasten).

Die Verbindung L2 wirkte selektiv aktiv gegen alle 3 Tumorlinien (d.h. nicht aktiv auf Fibroblasten) und war bei einer Verdünnung von 1:2000 noch aktiv.

**Tabelle 1. ED₅₀ (effektive Dosis bei der 50% der Humankrebszellen abgetötet wurden, Werte in µg/mL)**

| Verbindung | NCI460 | UACC62 | MCF7 | NCIADR |
|---|---|---|---|---|
| L1 | 25,0 | 45,0 | 25,0 | **8,0** |
| L2 * | **9,0** | **4,2** | **6,0** | **6,0** |

| | | | | |
|---|---|---|---|---|
| * Referenzbeispiel | | | | |

### Abbildung 3

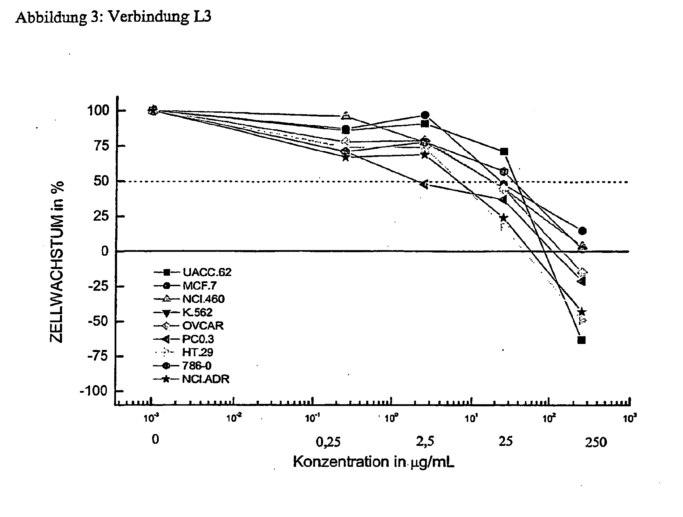

Die cytostatische und cytocide Aktivität gegen die gestesteten Zellinien, sowie die Selektivität der Verbindung L3 ist Abbildung 3 dargestellt

### Abbildung 4

Die Verbindung LP1 zeigte eine mittlere cytostatische Aktivität gegen alle getesteten Zell-Linien ab 25 µg/mL und cytocide Aktivität ab 250 µg/mL.

### Abbildung 5

Die Verbidung 2,40 zeigte eine mittlere cytostatische Aktivität gegen alle getesteten Zell-Linien ab 25 µg/mL und cytocide Aktivität ab 250 µg/mL.

### Abbildung 6

Die Verbindung LMK7 Referenzbeispiel zeigte cytostatische Aktivität gegen alle getesteten Zell-Linien ab 25 µg/mL, jedoch keine cytocide Aktivität gegen die getestete Lungenkrebszellinie.

### Antimikrobielle Tests (Minimale Hemmkonzentration, MHK)

Das Inokulum für die Tests wurde durch Verdünnung der Mikroorganismen in 0,85% NaCl hergestellt, die Verdünnung wurde auf Stufe 0,5 nach McFarland eingestellt und bei 580nm im Spectrophhotometer überprüft Die Zellsuspensionen wurden zur Verwendung in den Tests auf 10⁴ UFC/ml verdünnt Die MHK-Tests wurden nach der Vorschrift von J.N.P. Eloff (Planta Medica Vol. 64, pp. 711-713, 1998*))* in Mikrotiterplatten mit 96 Vertiefungen durchgeführt Serienverdünnungen der Konzentratione von 1,00-0,0015mg/ml wurden hergestellt Als Kontrollsubstanzen dienten Chloramphenicol bzw. Nistatin (Merck) in einer Konzentration von 0,062-0,005mg/ml bzw. 0,0125-0,001 mg/ml. Das Inokulum (100µl) wurde in alle Vertiefungen gegeben und die Platten für 48h bei 36°C inkubiert. Die antimikrobielle Aktivität wurde durch Zugabe von 20 µl einer 0,5% igen wässrigen Lösung von TTC (Tetrazoliumchlorid, Merck) pro Vertiefung detektiert Die minimale Hemmkonzentration (MHK) wurde als die niedrigste Konzentration der Verbindung definiert die sichtbares Wachstum hemmte (durch TTC Färbung angezeigt). Die Ergebnisse der antimikrobiellen Tests sind in Tabelle 2 gezeigte

**Tabelle 2. MHK-Werte (in mg/mL)**

| | | | | |
|---|---|---|---|---|
| Microorganismen | L1 | L2* | L3 | Vergleichsubstanz Chloramphenicol |
| *B. subitilis* | **0,5 -1,0** | **0,5-1,0** | > als 1,0 | 0,02 |
| *Micrococcus luteus* | **0,25-0,5** | **0,5 - 1,0** | **0,5 -1,0** | 0,05 |
| *Rhodococcus equi* | > als 1,0 | > als 1,0 | > als 1,0 | 0,04 |
| *E. falcium* | **0,25** | **0,5 - 1,0** | **0,5 -1,0** | 0,12 |
| *Salmonella choterasuis* | **0,25** | **0,5 -1,0** | > als 1,5 | 0,06 |
| *Escherichia coli* | > als 1,0 | **0,05** | > als 1,0 | 0,04 |
| *Candida albicans* | **0,06** | **0,05** | 0,9 | 0,05** |
| *Staphylococcus aureus* | **0,5** | > als 1,0 | > als 1,0 | 0,02 |

| | | | | |
|---|---|---|---|---|
| ** Vergleichsubstanz gegen *Candida albicans* Nistatin Referenzbeispiel | | | | |

**Tabelle 3. MIC-Werte in (mg/mL)**

| Microorganismen | **2,4O** | Vergleichsubtanz Chloramphenicol |
|---|---|---|
| *S*. *epidermides* | 0,5-1,0 | 0,04 |
| *E. coli* | 0,5-1,0 | 0,04 |
| *Rhodococcus equi* | 0,5-1,0 | 0,04 |
| *S. falcium* | 0,5-1,0 | 0,12 |
| *B. subtilis* | 0,5-1,0 | 0,02 |
| *Micrococcus, luteus* | 0,5-1,0 | 0,05 |
| *E. faecium* | 0,5-1,0 | 0,12 |
| *Salmonella* | 0,5-1,0 | 0,06 |
| *S. aureus* | 0,5-1,0 | 0,02 |
| *Candida albicans* | 0,5-1,0 | 0,12** |

| | | |
|---|---|---|
| ** Vergleichsubstanz gegen *Candida albicans* Nistatin | | |

**Tabelle 4. MIC-Werte in (mg/mL)**

| Microorganismen | LP1 | Vergleichsubstanz Chloramphenicol |
|---|---|---|
| *S. epidermides* | **0,06-0,12** | 0,04 |
| *E. coli* | > als 1,5 | 0,04 |
| *Rhodococcus equi* | > als 1,5 | 0,04 |
| *Candida albicans* | > als 1,5 | 0,12** |
| *B. subtilis* | > als 1,5 | 0,02 |
| *Micrococcus luteus* | > als 1,5 | 0,05 |
| *E.faecium* | > als 1,5 | 0,12 |
| *Salmonella* | **0,12-0,25** | 0,06 |
| *S. aureus* | > als 1,5 | 0,02 |
| *S. falcium* | > als 1,5 | 0,12 |

| | | |
|---|---|---|
| ** Vergleichsubstanz gegen *Candida albicans* Nistatin | | |

Die vorstehenden Testergebnissen zeigen die hervorragende antimikrobielle (gegen Bakterien und Pilze) antivirale bzw. antiproliferative Wirkung der erfindungsgemäßen Verbindungen und können somit zur Therapie und Prophylaxe von malignen Tumoren im Menschen wie z.B. soliden Tumoren wie Coloncarzinoma, Rektumkarzinoma, Magenkrebs, Krebs der Schilddrüse, Lungenkrebs, Blasenkrebs, Chorionkarzinom, Leberkrebs, Gebärmutterkrebs, Prostatakatzinom, Pharyngealkarzinom, Lungenkrebs, Mammakarzinom, malignes Melanom, Karposi Sarkom, Hirntumore, Neuroblastom, Ovarialkarzinom, Hodenkrebs, Osteosarkom, Pankreaskrebs, Nierenkrebs,Hypemephroma, und Angioendothelioma; und hematopoietische maligne Tumore wie Leukämie oder Lymphoma eingesetzt werden.

Ferner können die erfindungsgemässen Verbindungen als Arzneistoffe gegen Bakterien und Pilzinfektionen wie z.B. durch Candida albicans bei Soor, Pharyngitis, Interdigitalmykosen und gegen lokale und systemische Mykosen eingesetzt werden.

Zudem können die erfindungsgemsässen Verbindungen auch als immunstimuulierende bzw. immunmodulierende Wirkstoffe eingesetzt werden

Die vorliegende Erfindung stellt zudem auch Arzneimittel bereit die eine oder mindestens eine erfindungsgemäße Verbindung gegebenenfalls unter Beimischung von im Fachgebiet üblichen Hilfsstoffen und Exzipienten umfassen. Die erfindungsgemäßen Arzneimittel können nach üblichen dem Fachmann bekannten Verfahren und Techniken formuliert/hergestellt werden.

Bevorzugt sind hierbei Darreichungsformen zur oralen, parenteralen (z.B. i..v., s.c., i.p., i.c., intrathekal) und lokalen ( z. B. topisch, rectal, vaginal, buccal Applikation im Auge oder durch Inhalation) Applikation.

Somit können die erfindungsgemäßen Arzneimittel insbesondere als Tabletten (insbesondere auch magensaftresistent überzogene Tabletten oder Tabletten mit modifizierter Wirkstofffreigabe), Kapseln (Hart- und Weichgelatinekapseln), Pillen, Granulate, Suppositorien, Ovula, Salben Cremes, Gele, Pflaster , TTS oder auch als Emulsionen, Suspensionen, Lösungen oder rekonstituierbare Pulver (auch zur parenteralen Applikation) vorliegen.

## Patentansprüche

1. Hydroxyacetophenonderivat der allgemeinen Formel III: 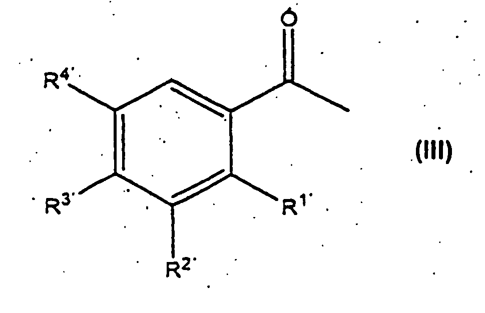 wobei R^{1'}, R^{2'} , R^{3'} und R^{4'} unabhängig voneinander ein Wasserstoffatom, einen Prenylrest, einen 1,1-Dimethylallylrest oder einen 3-Methylbut-2-enyloxyrest darstellen, mit der Maßgabe, dass mindestens ein Rest von R^{1'}, R^{2'}, R^{3'} und R^{4'}, ein Wasserstoffatom darstellt und dass mindestens ein Rest von R^{1'}, R^{2'}, R^{3'} und R^{4'}, ein 3-Methylbut-2-enyloxyrest ist, wobei 1-[2-(3-Methylbut-2-enyloxy)phenyl]ethanon, 1-[4-(3-Methylbut-2-enyloxy)phenyl]ethanon und 1-[2,4-Bis(3-methylbut-2-enyloxy)phenyl]ethanon ausgenommen sind.

2. Hydroxyacetophenonderivat gemäß Anspruch 1, umfassend:
1-[5-(3-Methylbut-2-enyl)-2-(3-methylbut-2-enyloxy)phenyl]ethanon,
1-[3-(1,1-Dimethylallyl)-2-(3-methylbut-2-enyloxy)phenyl]ethanon und
1-[5-(3-Methylbut-2-enyl)-4-(3-methylbut-2-enyloxy)phenyl]ethanon.

3. Arzneimittel, umfassend mindestens eine Verbindung ausgewählt aus einer Verbindung nach einem der Ansprüche 1 bis 2, 1-[2-(3-Methylbut-2-enyloxy)phenyl]ethanon, 1-[4-(3-Methylbut-2-enyloxy)phenyl]-ethanon und 1-[2,4-Bis(3-methylbut-2-enyloxy)phenyl]ethanon.

4. Verwendung mindestens einer Verbindung ausgewählt aus einer Verbindung nach einem der Ansprüche 1 bis 2, 1-[2-(3-Methylbut-2-enyloxy)phenyl]ethanon, 1-[4-(3-Methylbut-2-enyloxy)phenyl]ethanon und 1-[2,4-Bis(3-methylbut-2-enyloxy)phenyl]ethanon zur Herstellung eines antiproliferativen, antimikrobiellen (gegen Bakterien und Pilze), anti-viralen, immunmodulierenden oder immunstimulierenden Arzneimittels;

5. Verwendung nach Anspruch 4 zur Prophylaxe oder Therapie neoplastischer Erkrankungen ausgewählt aus Lungenkrebs, Mammakarzinom (normal und den MDR Phänotyp expremierend), Melanoma, Leukämie, Colonkarzinom, Nierenkarzinom, Ovarialkarzinom, Pankreaskrebs und Prostatakrebs.

6. Verwendung nach Anspruch 4 zur Phrophylaxe oder Therapie einer Infektion ausgelöst durch einen Mikroorganismus des Gattung Candida, Bacillus, Micrococcus, Rhodococcus, Escherichia, Enterococcus, Staphylococcus, Neisseria, Salmonella, Pseudomonas, Treponema, Mycobacterium oder Trichomonas

7. Verwendung nach Anspruch 6 zur Prophylaxe oder Therapie von einer Infektion, welche durch einen Microorganismus ausgewählt aus der Gruppe bestehend aus *Bacillus subtilis, Micrococcus luteus, Rhodococcus equi, Enterococcus faecium, Salmonella choterasuis, Escherichia coli, Candida albicans, Staphylococcus aureus und S. epidermides, S falcium, Neisseria gonorrhoeae, Salmonella pullorum, Pseudomonas aeruginosa, Treponema pallidum, Bacillus coagulans, Mycobacterium leprae, Trichomonas vaginalis, Bacillus cereus, Bacillus megaterium, Micrococcus roseus, Micrococcus varians, Salmonella typhi oder Mycobacterium tuberculosis,* verursacht wird.

## Claims

1. Hydroxyacetophenone derivative of the general formula III: 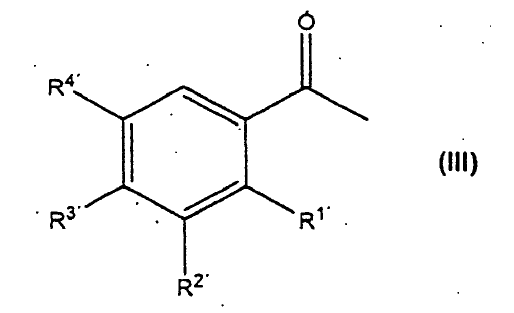 wherein R^{1'}, R^{2'}, R^{3'} and R^{4'} are independently from each other hydrogen, a prenyl group, a 1,1-dimethylallyl group or a 3-methylbut-2-enyloxy group with the proviso that at least one of R^{1'}, R^{2'}, R^{3'} and R^{4'} is hydrogen and that at least one of R^{1'}, R^{2'}, R^{3'} and R^{4'} is a 3-methylbut-2-enyloxy group, wherein 1-[2-(3-methylbut-2-enyloxy)phenyl]ethanone, 1-[4-(3-methylbut-2-enyloxy)phenyl]ethanone and 1-[2,4-bis(3-methylbut-2-enyloxy)phenyl]ethanone are excluded.

2. Hydroxyacetophenone derivative according to claim 1, comprising:
1-[5-(3-methylbut-2-enyl)-2-(3-methylbut-2-enyloxy)phenyl]ethanone,
1-[3-(1,1-dimethylallyl)-2-(3-methylbut-2-enyloxy)phenyl]ethanone and
1-[5-(3-methylbut-2-enyl)-4-(3-methylbut-2-enyloxy)phenyl]ethanone.

3. Pharmaceutical composition comprising at least one compound selected from a compound according to any one of claims 1 to 2, 1-[2-(3-methylbut-2-enyloxy)phenyl]ethanone, 1-[4-(3-methylbut-2-enyloxy)phenyl]ethanone and 1-[2,4-bis(3-methylbut-2-enyloxy)phenyl]ethanone.

4. Use of at least one compound selected from a compound according to any one of claims 1 to 2, 1-[2-(3-methylbut-2-enyloxy)phenyl]ethanone, 1-[4-(3-methylbut-2-enyloxy)phenyl]ethanone and 1-[2,4-bis(3-methylbut-2-enyloxy)phenyl]ethanone for the preparation of an antiproliferative, antimicrobial (against bacteria and fungi), antiviral, immune-modulating or immune-stimulating pharmaceutical composition.

5. Use according to claim 4 for the prophylaxis or therapy of neoplastic diseases selected from lung cancer, breast cancer (normal and expressing the MDR phenotype), melanoma, leukaemia, colon carcinoma, renal carcinoma, ovarian carcinoma, pancreas cancer and prostate cancer.

6. Use according to claim 4 for the prophylaxis or therapy of an infection caused by a microorganism of the genus Candida, Bacillus, Micrococcus, Rhodococcus, Escherichia, Enterococcus, Staphylococcus, Neisseria, Salmonella, Pseudomonas, Treponema, Mycobacterium or Trichomonas.

7. Use according to claim 6 for the prophylaxis or therapy of an infection, which is caused by a microorganism selected from the group consisting of *Bacillus subtilis, Micrococcus luteus, Rhodococcus equi, Enterococcus faecium, Salmonella choterasuis, Escherichia coli, Candida albicans, Staphylococcus aureus and S. epidermides, S. falcium, Neisseria gonorrhoeae, Salmonella pullorum, Pseudomonas aeruginosa, Treponema pallidum, Bacillus coagulans, Mycobacterium leprae, Trichomonas vaginalis, Bacillus cereus, Bacillus megaterium, Micrococcus roseus, Micrococcus varians, Salmonella typhi or Mycobacterium tuberculosis.*

## Revendications

1. Dérivé d'hydroxyacétophénone de formule générale III : 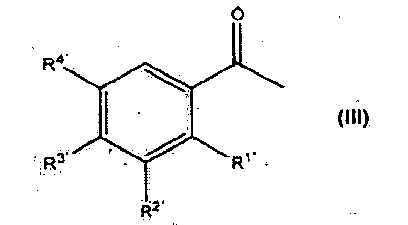 dans laquelle R^{1'}, R^{2'}, R^{3'} et R^{4'}, indépendamment les uns des autres, représentent un atome d'hydrogène, un résidu prényle, un résidu 1,1-diméthylallyle ou un résidu 3-méthylbut-2-ènyloxy, à la condition qu'au moins un résidu parmi R^{1'}, R^{2'}, R^{3'} et R^{4'} représente un atome d'hydrogène et qu'au moins un résidu parmi R^{1,} R^{2'}, R^{3'} et R^{4'} représente un résidu 3-méthylbut-2-ènyloxy, la 1-[2-(3-méthylbut-2-ènyloxy)phényl]-éthanone, la 1-[4-(3-méthylbut-2-ènyloxy)phényl]-éthanone et la 1-[2,4-bis(3-méthylbut-2-ènyloxy)phényl]-éthanone en étant exclues.

2. Dérivé d'hydroxyacétophénone selon la revendication 1, comprenant :
la 1-[5-(3-méthylbut-2-ènyl)-2-(3-méthylbut-2-ènyloxy)phényl]-éthanone,
la 1-[3-(1,1-diméthylallyl)-2-(3-méthylbut-2-ènyloxy)phényl]-éthanone et
la 1-[5-(3-méthylbut-2-ènyl)-4-(3-méthylbut-2-ènyloxy)phényl]-éthanone.

3. Médicament comprenant au moins un composé choisi parmi un composé selon l'une des revendications 1 à 2, la 1-[2-(3-méthylbut-2-ènyloxy)phényl]-éthanone, la 1-[4-(3-méthylbut-2-ènyloxy)phényl]-éthanone et la 1-[2,4-bis(3-méthylbut-2-ènyloxy)phényl]-éthanone.

4. Utilisation d'au moins un composé choisi parmi un composé selon l'une des revendications 1 à 2, la 1-[2-(3-méthylbut-2-ènyloxy)phényl]-éthanone, la 1-[4-(3-méthylbut-2-ènyloxy)phényl]-éthanone et la 1-[2,4-bis(3-méthylbut-2-ènyloxy)phényl]-éthanone pour la préparation d'un médicament antiprolifératif, antimicrobien (contre les bactéries et les champignons), antiviral, immunomodulateur ou immunostimulant.

5. Utilisation selon la revendication 4 pour la prophylaxie ou la thérapie des affections néoplasiques choisies parmi le cancer du poumon, le carcinome mammaire (normal et exprimant le phénotype MDR), le mélanome, la leucémie, le carcinome du colon, le carcinome rénal, le carcinome ovarien, le cancer du pancréas et le cancer de la prostate.

6. Utilisation selon la revendication 4 pour la prophylaxie ou la thérapie d'une infection déclenchée par un micro-organisme du genre Candida, Bacillus, Micrococcus, Rhodococcus, Escherichia, Enterococcus, Staphylococcus, Neisseria, Salmonella, Pseudomonas, Treponema, Mycobacterium ou Trichomonas.

7. Utilisation selon la revendication 6 pour la prophylaxie ou la thérapie d'une infection, qui est provoquée par un micro-organisme choisi dans le groupe constitué par *Bacillus subtilis, Micrococcus luteus, Rhodococcus equi, Enterococcus faecium, Salmonella choterasuis, Escherichia coli, Candida albicans, Staphylococcus aureus* et *S*. *epidermides, S. falcium, Neisseria gonorrhoeae, Salmonella pullorum, Pseudomonas aeruginosa, Treponema pallidum, Bacillus coagulans, Mycobacterium leprae, Trichomonas vaginalis, Bacillus cereus, Bacillus megaterium, Micrococcus roseus, Micrococcus varians, Salmonella typhi,* ou *Mycobacterium tuberculosis.*
